## Europäisches Patentamt

## European Patent Office

## Office européen des brevets

(11) Veröffentlichungsnummer: **0 263 529**
A1

(12)

# EUROPÄISCHE PATENTANMELDUNG

(21) Anmeldenummer: 87114793.0

(51) Int. Cl.4: **C07K 7/06 , C07K 7/08**

(22) Anmeldetag: 09.10.87

(30) Priorität: 09.10.86 US 916773
13.11.86 US 930588
10.08.87 US 83322

(43) Veröffentlichungstag der Anmeldung:
**13.04.88 Patentblatt 88/15**

(84) Benannte Vertragsstaaten:
**AT BE CH DE FR GB IT LI LU NL SE**

(71) Anmelder: **F. HOFFMANN-LA ROCHE & CO.
Aktiengesellschaft**

**CH-4002 Basel(CH)**

Anmelder: **Oklahoma Medical Research
Foundation
825 N.E. 13th Street
Oklahoma City Oklahoma 73104(US)**

(72) Erfinder: **Felix, Arthur M.
257 Central Avenue
West Caldwell, N.J.07006(US)**
Erfinder: **Heimer, Edgar P.
87 Edison Terrace
Sparta, N.J. 07871(US)**
Erfinder: **Nawroth, Peter P.
Heydornweg 10
D-2000 Hamburg(DE)**
Erfinder: **Stern, David M.
63 Tanners Road
Great Neck, N.Y. 11020(US)**
Erfinder: **Wilner, George D.
2318 Whittemore Place
St.Louis, Missouri 63104(US)**

(74) Vertreter: **Lederer, Franz, Dr. et al
Patentanwält Dr. Franz Lederer Lucile
Grahnstrasse 22
D-8000 München 80(DE)**

(54) **Faktor-IX-Peptide.**

(57) Die Erfindung betrifft Polypeptide, die die EGF-Domäne des Faktors IX oder eine Untersequenz davon enthalten und die zur spezifischen Hemmung der Bindung von Gerinnungsfaktor IX bzw. dessen aktivierter Form IXa an Endothelzelloberflächen geeignet sind. Ferner betrifft die Erfindung pharmazeutische Präparate die solche Polypeptide enthalten. Die Polypeptide und die pharmazeutischen Präparate eröffnen einen neuen Zugang zur Behandlung thrombotischer Erkrankungen, bei der die schützende extravaskuläre Gerinnung nicht beeinträchtigt wird, die die folgenden Aminosäuresequenzen umfassen:

Asp-Gly-Asp-Gln-Cys,
Lys-Gln-Tyr-Val-Asp-Gly-Asp-Gln-Cys,
Phe-Trp-Lys-Gln-Tyr-Val-Asp-Gly-Asp-Gln-NH2,
Lys-Gln-Tyr-Val-Asp-Gly-Asp-Gln-Cys-Glu-Ser-Asn-NH2 und
Lys-Gln-Tyr-Val-Asp-Gly-Asp-Gln-NH2.

## Faktor-IX-Peptide

Die vorliegende Erfindung bezieht sich auf neue Polypeptidantagonisten des Gerinnungsfaktors IXa. Die Bindung dieser Antagonisten an die zellulären Rezeptoren des Gerinnungsfaktors IXa kann selektiv vaskuläre Thrombose verhindern ohne die schützende, extravaskuläre Gerinnung zu beeinträchtigen, wodurch ein neuer Zugang zur Behandung thrombotischer Erkrankungen zur Verfügung gestellt wird.

Faktor IX ist ein Vitamin K-abhängiges Plasmaglykoprotein mit 416 Aminosäuren, das eine zentrale Rolle im Koagulationsmechanismus spielt [Fujikawa et al., Biochem. 12, 4938-4945 (1973); Katayama et al., Proc. Natl. Acad. Sci. USA 76, 4990-4994 (1979); Choo et al., Nature 299, 178-180 (1982); Kurachi et al., Proc. Natl. Acad. Sci. USA 79, 6461-6464 (1982) und Jaye et al., Nucl. Acids Res. 11, 2325-2335 (1983)]. Die vollständige Aminosäuresequenz des Faktors IX des Menschen und des Rinds sind veröffentlicht worden [Katayama et al., Choo et al., Kurachi et al. und Jaye et al., siehe oben]. Nach Aktivierung durch limitierte Proteolyse durch Faktor XIa [Fujikawa et al., Biochem. 13 , 4508-4516 (1974)] oder Gewebsfaktor VIIa [Østerud et al., Proc. Natl. Acad. Sci. USA 74, 5260-5264 (1977)] unterstützt Faktor IXa, die enzymatisch aktive Form von Faktor IX, die Aktivierung von Faktor X in Gegenwart von Faktor VIII, Calcium und einer geeigneten zellulären-oder Phospholipidoberfläche [van Dieijen et al., J. Biol. Chem. 256, 3433-3442 (1981)-].

Obwohl Faktor IX ein wichtiges Koagulationsprotein in der Hämostase (der Verhinderung von Blutungen) darstellt, wurde es auch mit der Thrombose, der pathologischen Bildung von intravaskulären Gerinseln, die Blutgefässe verschliessen und Herzinfarkte, Schlaganfälle und andere Krankheiten, bei denen thrombolytische Mechanismen eine Rolle spielen, zur Folge haben, in Verbindung gebracht. So hat Infusion von Faktor IXa enthaltender Lösung bei Kaninchen lokale Thrombosen im Wesslerschen Venostase-Modell zur Folge [Gitel et al., Proc. Natl. Acad. Sci. USA 74, 3028-3032 (1977)]. Faktor IXa stellt, im Vergleich zu anderen Koagulationsproteinen, das stärkste Thrombogen dar.

Neue Studien haben sich vor allem mit der Wechselwirkung von Faktor IX mit Endothelzellen als Grundlage für lokale Gerinselbildung beschäftigt [Stern et al., Proc. Natl. Acad. Sci. USA 80, 4119-4123 (1983); Heimark et al., Biochem. Biophys. Res. Comm. 111, 732-731 (1983); Stern et al., J. Clin. Invest. 74, 1910-1921 (1984); Stern et al., Proc. Natl. Acad. Sci. USA 81, 913-917 (1984); Stern et al.,‘ F. Biol. Chem. 260, 6717-6722 (1985); Nawroth et al., Brit. J. Haematol. 63, 309-320 (1986)]. Da das Endothel, also die Zellen mit denen die Blutgefässe ausgekleidet sind, eine allgegenwärtige, vaskuläre Oberfläche darstellt, liegt die Vermutung nahe, dass die Wechselwirkung von Faktor IXa mit seiner Bindungsstelle an den Endothelzelloberflächen eine Grundlage der Thrombogenität des Faktors IXa sein könnte.

Die vorliegende Erfindung stellt Methoden und Kompositionen zur selektiven Hemmung der Bindung des Blutge rinnungsfaktors IX und seiner aktiven Form (Faktor IXa) an sein zellulären Rezeptoren im vaskulären Endothelium zur Verfügung. Die Hemmung der Bindung von Faktor IX/IXa wird durch die Verwendung neuer, von einer bestimmten Region des Faktor IX-Proteins abgeleiteter, Polypeptide erreicht.

Es wurde gefunden, dass Peptide, die die epidermale Wachstumsfaktor (EGF)-Domäne des Faktor IX-Moleküls oder Subsequenzen davon enthalten, mit dem Faktor IX/IXa in spezifischer Weise um die Bindung an seine zellulären Rezeptoren konkurrieren können, wodurch die Bindung von Faktor IX/IXa inhibiert wird. Durch die Hemmung dieser Bindung kann vaskuläre Thrombose verhindert werden. Da die spezifische Wirkungsweise der erfindungsgemässen Peptide auf das vaskuläre Endothel begrenzt ist, kann vaskuläre Thrombose ohne Beeinträchtigung der normalen extravaskulären Gerinnung, die wesentlich ist zur Behebung von Verletzungsfolgen, verhindert werden.

Die Funktion der EGF-Domäne in Faktor IX-Molekül ist nicht bekannt. Ihr Name folgt aus der Tatsache, dass sie eine gewisse Homologie zu EGF, einem Polypeptidhormon von dem bekannt ist, dass es über zelluläre Rezeptoren verfügt, zeigt. Die EGF-Domäne umfasst die Aminosäurereste 47-136 im Faktor IX-Molekül.

Im Gegensatz zu anderen Antikoagulantien, wie Gewebe-Plasminogenaktivator (TPA), beeinflussen die erfindungsgemässen Peptide die Blutgerinnung in vitro nicht. So können im Gegesatz zur fibrinolytischen Therapie (z.B. mittels TPA), die bereits gebildete Gerinsel aufzulösen sucht, die Verbindungen der vorliegenden Erfindung in erster Linie präventiv verwendet werden, d.h. um die Bildung gefährlicher vaskulärer Gerinsel zu verhindern. Ferner können die Methoden und Kompositionen der Erfindung zur Nachfolge-und stabilisierenden Therapie verwendet werden, um Gerinselneubildung, nach erfolgter Beseitigung vorhandener Gerinsel durch chirurgischen Eingriff, mittels fibrinolytischer Therapie (z.B. mit TPA) oder durch andere Methoden, zu verhindern.

Gegenstand der vorliegenden Erfindung sind daher neue Peptide, die die EGF-Domäne des Faktors IX oder Subsequenzen davon enthalten und die zur selektiven Bindung an die zellulären Rezeptoren des Faktors IX/IXa befähigt sind. Beispiele erfindungsgemässer Peptide sind solche, die die folgenden Aminosäuresequenzen umfassen:

Asp-Gly-Asp-Gln-Cys,

Lys-Gln-Tyr-Val-Asp-Gly-Asp-Gln-Cys,

Phe-Trp-Lys-Gln-Tyr-Val-Asp-Gly-Asp-Gln-NH₂,

Lys-Gln-Tyr-Val-Asp-Gly-Asp-Gln-Cys-Glu-Ser-Asn-NH₂ und

Lys-Gln-Tyr-Val-Asp-Gly-Asp-Gln-NH₂.

Diese Sequenzen entsprechen den Aminosäureresten 47-51, 43-51, 41-50, 43-54 und 43-50 der Faktoren IX von Mensch und Rind.

Gegenstand der Erfindung sind weiterhin pharmazeutische Zusammensetzungen auf der Basis eines Peptids, das die EGF-Domäne des Faktors IX oder eine Subsequenz davon enthält und das zur selekiven Bindung an die zellulären Rezeptoren des Faktors IX/IXa befähigt ist, und pharmazeutisch sinnvoller Trägermaterialien.

Gegenstand der Erfindung sind weiterhin Methoden zur Verhinderung von Thrombosen, die dadurch charakterisiert sind, dass man einem Tier oder Menschen eine therapeutisch wirksame Menge eines Peptids, das die EGF-Domäne des Faktors IX oder eine Subsequenz davon enthält und das zur selektiven Bindung an die zellulären Rezeptoren des Faktors IX/IXa befähigt ist, zusammen mit einem pharmazeutisch sinnvollen Trägermaterial appliziert.

Die Peptide der voliegenden Erfindung können mittels konventioneller Methoden der Peptidsynthese, in flüssiger oder, vorzugsweise an fester Phase, hergestellt werden, z.B. mit der Methode von Merrifield [J. Am. Chem. Soc. 85, 2149-2154 (1963)] oder einer anderen gleichwertigen Methode des Standes der Technik.

Die Festphasensynthese beginnt mit der C-terminalen Aminosäure des zu synthetisierenden Pepids, die in geschützter Form an ein passendes Trägermaterial gekoppelt wird. Das Ausgangmaterial kann durch Bindung einer aminogruppengeschützten Aminosäure über eine Benzylesterbrücke an chlormethyliertes oder hydroxymethyliertes Trägermaterial oder durch Amidbindung an ein Benzylhydrylamin (BHA)-oder Methylbenzylhydrylamin (MBHA)-Trägermaterial hergestellt werden. Diese Trägermaterialeien sind käuflich erhältlich und ihre Bereitstellung und Verwendung sind wohlbekannt.

Allgemeine Methoden zum Schützen und zum Entfernen der Schutzgruppen von Aminosäuren, die in dieser Erfindung verwendet werden können, sind beschrieben in "The Peptides", Vol. 2 [herausgegeben von E. Gross und J. Meienhofer, Academic Press, New York, 1-284 (1979)]. Schutzgruppen umfassen z.B. die tertiäre Butyloxycarbonyl (Boc)-, die Benzyl (Bzl)-, die 2-Chlorbenzyloxycarbonyl (2Cl-Z)-und die 3,4-Dimethylbenzyl-(Dumb)-gruppe.

Nach Entfernen der α-Aminoschutzgruppe der an den Träger gebundenen C-terminalen Aminosäure werden die übrigen geschützten Aminosäuren in der gewünschten Reihenfolge schrittweise angekoppelt. Das vollständige Peptid kann so synthetisiert werden. Als Alternative dazu können kleine Pepiide aufgebaut werden, die dann zum gewünschten Peptid zusammengefügt werden. Geeignete Kopplungsreagenzien gehören zum Stand der Technik, wobei Dicyclohexylcarbodiimid (DCC) besonders geeignet ist.

Jede(s) geschützte Aminosäure oder Peptid wird im Ueberschuss in die Festphasensynthesemaschine gegeben und die Kopplungsreaktion kann in Dimethylformamid (DMF), in Methylenchlorid (CH₂Cl₂) oder einer Mischung aus beiden durchgeführt werden. In Fällen von unvollständiger Kopplung wird die Kopplungsreaktion wiederholt bevor die N-terminale α-Aminoschutzgruppe zwecks Kopplung der nächsten Aminosäure entfernt wird. Die Ausbeute jedes Kopplungsschrittes kann verfolgt werden und zwar bevorzugt nach der Ninhydrinmethode. Die Kopplungsreaktionen und die Waschschritte können automatisiert durchgeführt werden.

Die Abspaltung des Peptids vom Trägermaterial kann durch in der Peptidchemie wohlbekannte Methoden erreicht werden, z.B. durch Umsetzung mit Fluorwasserstoff (HF) in Gegenwart von p-Kresol und Dimethylsulfid während 1 Stunde bei 0°C, möglicherweise gefolgt von einer zweiten Umsetzung mit HF in Gegenwart von p-Kresol während 2 Stunden bei 0°C. Die Abspaltung der Peptide von chlormethylierten oder hydroxymethylierten Trägermaterialien ergibt Peptide mit freiem C-Terminus; die Abspaltung der Peptide von Benzylhydrylamin-oder Methylbenzylhydrylamin-Trägern ergibt Peptide mit amidiertem C-Terminus. Im vorliegenden Text sind derartige Reste dargestellt als -X-NH₂, wobei X für die C-terminale Aminosäure steht.

Die Peptide der vorliegenden Erfindung können aber auch mittels bekannter Methoden der DNA-Technologie hergestellt werden.

3

Die erfindungsgemässen Polypeptide können in bekannter Weise gereinigt werden, beispielsweise durch Gelfiltration, isoelektrische Fokussierung, Ionenaustausch-und Verteilungs-oder Gegenstromverteilungschromatographie. Präparative HPLC wird bevorzugt verwendet.

Die erfindungsgemässen Peptide können in Form von human-oder veterinärmedizinischen Präparaten, die mittels üblicher, pharmazeutischer Formulierungstechniken hergestellt werden, angewendet werden und zwar oral, intravenös, subcutan,intranasal, intramuskulär oder intraperitoneal. Für Patienten, die fibrinolytisch therapiert werden, ist die intravenöse Infusion die Applikationsweise der Wahl. Wo kleinere Peptide Anwendung finden, können dünndarmlösliche oder kunststoffverkapselte, zur oralen Applikation geeignete Präparate hergestellt werden.

Die erfindungsgemässen Peptide können als Lyophilisate zur Rekonstitution mit sterilem Wasser oder Kochsalzlösung vorliegen. Die am besten geeignete Dosierungsform hängt von dem zu behandelnden Patienten und den Bedingungen der Verwendung ab, und ist für einen Durchschnittsfachmann offensichtlich. Ausschliesslich Routineversuche werden benötigt, um die geeignete Dosis für den human-bzw. tiermedizinischen Fall zu bestimmen.

Die ersten 15 Aminosäurereste der EGF-Domäne (entsprechend den Resten 46-60 des Faktors IX) sind im Faktor IX des Menschen und des Rinds identisch. Daneben gibt es einige Unterschiede zwischen entsprechenden Resten der beiden Moleküle, obwohl die Sequenzen sonst weitgehend homolog sind. Von den grösseren Peptiden, die weitgehend oder vollständig die ganze EGF-Domäne umfassen, oder den kleinen Peptiden, die Aminosäuresubsequenzen variable Regionen entsprechen, werden für die Humantherapie solche auf der Grundlage der menschlichen Sequenz [Kurachi et al., Proc. Natl. Acad. Sci. USA 79, 6461-6464 (1982)] bevorzugt verwendet.

Nichtsdestoweniger ist die Homologie zwischen den Faktoren IX verschiedener Säugetierspezies solcherart, dass, wie man anhand der folgenden Beispiele sehen kann, erfindungsgemässe Peptide auf der Basis von EGF-Sequenzen von Mensch und Rind, an vaskulärem Gewebe von Rind und Kaninchen wirksam waren.

Die Erfindung wird durch die folgenden Beispiele in nichteinschränkendem Sinn weiter veranschaulicht.

Beispiel 1

Synthese und Reinigung der Peptide

Die folgenden Abkürzungen werden in der folgenden Beschreibung verwendet:

BHA = Benzylhydrylamin
Boc = tertiär Butyloxycarbonyl
Bzl = Benzyl
2Cl-Z = 2-Chlorbenzyloxycarbonyl
Dmb = 3,4-Dimethylbenzyl
Dcb = Dichlorbenzyl

Ein Pentapeptid und ein Nonapeptid, entsprechend den Aminosäureresten 47-51 udn 43-51 des Faktors IX vom Menschen und Rind, wurden nach einer von Wilner et al. [Biochem. 15, 1209-1213 (1976)] beschriebenen Modifikation der Synthese an fester Phase nach Merrifield synthetisiert. Diey Synthese wurde an einem mikrocomputergesteuerten Vega 1000-Syntheseautomaten durchgeführt. Die Abspaltung vom Träger und die Entfernung der Schutzgruppen des vollständig geschützten Peptids erfolgt mittels wasserfreier HF nach der Zwei-Schritt-Methode von Tam et al. [J. Am. Chem. Soc. 105, 6442-6455 (1983)].

Die Reinigung der ungeschützten Peptide wurde mittels Hochdruckflüssigkeitschromatographie (HPLC) an einer Spherisorb ODS-1 (2,5 x 25 cm)-Säule, die mit einem 0,025% Trifluoressigsäure (TFA)-Wasser-Acetonitril-Gradienten eluiert wurde, durchgeführt. Die Reinheit der Produkte betrug mehr als 95% gemäss analytischer HPLC. Die Amino säurezusammensetzungen wurden durch Aminosäureanalyse bestätigt.

Die weitere Reinigung der oben genannten Peptide zur Homogenität wurde mittels einer Nucleosil $C_{18}$ - (5 $\mu$)-Säule (1 x 50 cm), die mit einem Lösungsmittelsystem aus Wasser und $CH_3CN$ (beide mit 0,025% TFA) und einem linearen Gradienten von 5-25% $CH_3CN$ in 140 Minuten bei einer Durchflussrate von 3 ml/min eluiert wurde, durchgeführt. 3 ml-Fraktionen wurden gesammelt und Aliquote wurden mittels analytischer HPLC analysiert. Die die Peptide enthaltenden Fraktionen wurden gesammelt, verdampft und lyophilisiert.

Die Aminosäuresequenzen der gereinigten Peptide lauteten: Asp-Gly-Asp-Gln-Cys und Lys-Gln-Tyr-Val-Asp-Gly-Asp-Gln-Cys.

Ein Dodecapeptid, entsprechend den Aminosäureresten 43-54 des Faktors IX des Menschen und des Rinds, wurde wie folgt hergestellt: Boc-Asn-BHA-Trägermaterial (5 g, 0,20 meq/g-Trägermaterial) wurde in das Reaktionsgefäss eines Syntheseautomaten (Modell Vega Coupler 1000) gegeben und die Festphasensynthese wurde nach dem symmetrischen Anhydridverfahren mittels der von Barnay et al. beschriebenen Methode ["The Peptides: Analysis, Synthesis, Biology", herausgegeben von E. Gross und J. Meienhofer, Vol. 2, Academic Press, New York, 1-284 (1979)] durchgeführt. Um das vollständige, geschütze, an die Festphase gebundene Peptid zu erhalten, wurden 11 Zyklen mit folgenden N$\alpha$-Boc-Aminosäuren: Ser(Bzl), Glu(OBzl), Cys(Dmb), Gln, Asp(OBzl), Gly, Asp(OBzl), Val, Tyr(Dcb), Gln und Lys(2Cl-Z) durchgeführt.

Ein Teil des geschützten und an das Harz gekoppelten Peptids (0,4 g) wurde mit wasserfreier, 10% Dithioethan enthaltender HF 1 Stunde bei 0°C behandelt. Die HF wurde bei 0°C abgezogen (Hockvakuum, CaO-Falle) und die rohe Mischung aus Peptid und Matrix wurde mit EtOAc behandelt, mit TFA extrahiert, TFA wurde abgezogen und der Rückstand mit Ether behandelt und getrocknet. Die Ausbeute betrug 0,19 g.

Das Rohmaterial wurde in 10 ml 0,025% TFA enthaltendem Wasser gelöst, durch einen 0,45 $\mu$ Millipore Type HA Filter filtriert und wie oben beschrieben in der Nucleosil C$_{18}$-Säule gereinigt. Die endgültige Ausbeute betrug 12 mg. Das Produkt war nach analytischen HPLC-Daten homogen und verfügte über die erwartete Aminosäurezusammensetzung (6N HCl, 1% TFA, 150°C, 1 Stunde): Asp 2,93; Ser 0,95; Glu 3,03; Gly 1,10; Val 1,05; Tyr 0,91; Lys 1,00. Sequenzanalyse bestätigte die korrekte Aminosäuresequenz und "Fast atom bombardment"-Massenspektroskopie ergab eine Masse von 1384,26 (MH$^+$) [berechneter Wert: 1384.58 (MH$^+$)].

Die Aminosäuresequenz des Peptids ist: Lys-Gln-Tyr-Val-Asp-Gly-Asp-Gln-Cys-Glu-Ser-Asn-NH$_2$.

In analoger Weise wurden die Säureamide der Peptide aus den Aminosäureresten 43-50, 91-106 und 113-124 des Faktors IX des Rinds hergestellt. Die Aminosäuresequenzen dieser Peptide sind:

Lys-Gln-Tyr-Val-Asp-Gly-Asp-Gln-NH$_2$,

Phe-Trp-Lns-Gln-Tyr-Val-Asp-Gly-Asp-Gln-NH$_2$,

Lys-Asn-Gly-Arg-Cys-Lys-Gln-Phe-Cys-Lys-Arg-Asp-Thr-Asp-Asn-Lys-NH$_2$ und

Asp-Gly-Tyr-Arg-Leu-Ala-Glu-Asp-Gln-Lys-Ser-Cys-NH$_2$.

Die Aminosäuresequenz des Octapeptids entspricht den Aminosäuren 1-8 des vorstehend erwähnten Nonapeptids. Das Decapeptid enthält die Aminosäuresequenz des Octapeptids und zwei zusätzliche Aminosäuren am N-Terminus.

Das Dodecapeptid und das Hexadecapeptid wurden nach einer computergestützten Abschätzung der Sequenz des Faktors IX und zwar in der Weise hergestellt, dass beide Peptide bestimmten Regionen des Faktors IX, die auf der Oberfläche des Moleküls liegen, entsprechen. Zur computergestützten Analyse wurden Programme auf der Grundlage der Arbeiten von Hopp et al. [Proc. Natl. Acad. Sci. USA, 78, 3824-3828 (1981)], Garnier et al. [J. Mol. Biol. 120, 97-120 (1978)] und Kyte et al. [J. Mol. Biol. 157, 105-132 (1982)] verwendet.

Analysen der Aminosäurezusammensetzungen dieser vier Peptide bestätigten die erwarteten Werte und für alle Peptide konnte mittels analytischer HPLC Homogenität gezeigt werden.

Beispiel 2

Aktivitäten der Peptide

In vitro Bindungsstudien : Die gereinigten Peptide wurden auf ihre Fähigkeit getestet, die Bindung von Rinder-Faktor IX und IXa an kultivierten Rinderaortaendothelzellen zu blockieren. Rinderfaktor IX und IXa wurde für diese Studien hergestellt und mittels der bei Stern et al. [J. Biol. Chem. 260, 6717-6722 (1985)] beschriebenen Methoden mit radioaktivem Jod markiert (mittels Na$^{125}$I und der Festphasen-Lactoperoxidase-Technik). Rinderaortaendothelzellen wurden von den Aorten von neugeborenen Kälbern, wie bei Stern et al. [Proc. Natl. Acad. Sci. USA 80, 4119-4123 (1983)] beschrieben, erhalten und bis zur Bildung eines geschlossenen Zellrasens (1,3 x 10$^5$ Zellen/cm$^2$) in Kulturschalen herangezogen. Bindungsnachweise wurden durch Inkubation von [$^{125}$I]-Faktor IX oder [$^{125}$I]-Faktor IXa mit Einzelschichten von Endothelzellen, in Gegenwart unterschiedlicher Konzentrationen eines der erfindungsgemässen Peptide, durchgeführt. Als Puffer wurde 0,5 mg/ml Transferrin enthaltendes Minimalmedium (MEM) verwendet und es wurde 4-5 Stunden bei 4°C inkubiert. Nach dem Waschen der Kulturen am Ende der inkubationszeit (um nichtgebundenes Material zu entfernen) wurde spezifisch gebundener [$^{125}$I]-Faktor IX bzw. [$^{125}$I]-Faktor IXa mittels EDTA-enthaltendem Puffer, wie bei Stern et al. [Proc. Natl. Acad. Sci. USA 80, 4119-4123 (1983)] beschrieben, eluiert. Inhibierung der Bindung von [$^{125}$I]-Faktor IX wurde beobachtet (Tabelle 1).

## Tabelle 1*

## Inhibierung der Bindung von $[^{125}I]$-Faktor IX

| Inhibitor | Inhibitorkonstante | |
|---|---|---|
| Faktor IX | 3 | nM |
| Faktor IXa | 2.5 | nM |
| Asp-Gly-Asp-Gln-Cys | 90 | µM |
| Lys-Gln-Tyr-Val-Asp-Gly-Asp-Gln-Cys | 100 | µM |
| Phe-Trp-Lys-Gln-Tyr-Val-Asp-Gly-Asp-Gln-NH$_2$ | 110 | µM |
| Lys-Gln-Tyr-Val-Asp-Gly-Asp-Gln-Cys-Glu-Ser-Asn-NH$_2$ | 90 | µM |
| Lys-Gln-Tyr-Val-Asp-Gly-Asp-Gln-NH$_2$ | 105 | µM |
| Asp-Gly-Tyr-Arg-Leu-Ala-Glu-Asp-Gln-Lys-Ser-Cys-NH$_2$ | >1 | mM |
| Lys-Asn-Gly-Arg-Cys-Lys-Gln-Phe-Cys-Lys-Arg-Asp-Thr-Asp-Asn-Lys-NH$_2$ | >1 | mM |

* Kompetitive Bindungsstudien wurden, wie im Text beschrieben, durchgeführt. Die Inhibitorkonstante stellt den Mittelwert experimentell bestimmter Werte dar. Die Standardfehler des Mittelwerts betragen weniger als 20%.

Das Dodecapeptid, das den Resten 43-54 des Rinderfaktors IX entspricht, das Decapeptid, das Nonapeptid, das Octapeptid und das Pentapeptid zeigten ungefähr gleiche Wirksamkeit, aber deutlich geringere als der Faktor IX/IXa. Das andere Dodecapeptid und das Hexadecapeptid waren bis zu Konzentrationen von 1 mM völlig unwirksam.

Die Spezifität der Inhibierung der Bindung des $[^{125}I]$-Faktor IX durch die aktiven Peptide der EGF-Domäne wurde in Studien unter Verwendung von Fibrinopeptid A (Ala-Asp-Ser -Gly-Glu-Gly-Asp-Phe-Leu-Ala-Glu-Gly-Gly-Val-Arg), Fibrinopeptid B (PGA-Gly-Val-Asn-Asp-Asn-Glu-Glu-Gly-Phe-Phe-Ser-Ala-Arg) und Angiotensin I (Asp-Arg-Val-Tyr-Ile-His-Pro-Phe-His-Leu) gezeigt. Mit bis zu millimolaren Mengen der letzten 3 der genannten Peptide wurde keine Inhibierung der Bindung von $[^{125}I]$-Faktor IX an Endothelzellen beobachtet. Auch das Pentapeptid Glu-Gly-Glu-Gln-Cys, in dem die Asparaginsäurereste der ursprünglichen Sequenz durch Glutaminsäurereste ersetzt worden waren, war als Inhibitor unwirksam.

Diese Daten deuten daraufhin, dass es sich bei der Inhibierung der Bindung von Faktor IX/IXa an Endothelzellen durch die erfindungsgemässen Peptide um einen spezifischen Effekt handelt. Im Gegensatz zu der Inhibierung der Bindung des Faktor IX, hatte keines der Peptide einen inhibitorischen Effekt auf die Bindung von radioaktiv markiertem Faktor X an seine Endothelbindungsstellen. Die Bindung von Faktor X an die Oberfläche von Endothelzellen war kürzlich gezeigt worden [siehe Heimark et al., Biochem. Biophys. Res. Com. 111, 723-731 (1983); Stern et al., Proc. Natl. Acad. Sci. USA 81, 913-917 (1984)]. Da Faktor X einen weiteren Vitamin K-abhängigen Koagulationsfaktor darstellt, wird die Spezifität der Wirkung der erfindungsgemässen Peptide durch deren Unfähigkeit mit dem Faktor X zu konkurrieren, verdeutlicht.

In vitro Aktivierungsstudien: Zusätzlich zu den Faktor IX/IXa-Bindungsstudien, wurden kinetische Experimente durchgeführt, um die Wirkung der Peptide auf die Endothelzellen-abhängige, durch die Faktoren IXa-VIII vermittelte Aktivierung von Faktor X abschätzen zu können. Für diese Studien wurden die Faktoren IXa und X nach Standardmethoden gereinigt. Faktor VIII (Europäische Patentanmeldung Nr. 160457 veröffentlicht am 6.11.1985) wurde grosszügigerweise von der Firma Genentech San Francisco-Süd, CA, USA) zur Verfügung gestellt.

Einzellschichten von Endothelzellen wurden gemischt und mit steigenden Konzentrationen eines erfindungsgemässen Peptids in Gegenwart der Faktoren IXa, VIII und X inkubiert. Die Produktbildungsrate (Faktor Xa-Bildung) wurde, unter Verwendung eines chromogenen Substrates, mittels eines von van Dieijen et al. [J. Biol. Chem. 256, 3433-3422 (1981)] beschriebenen Nachweises bestimmt. Die Ergebnisse sind in Tabelle 2 gezeigt.

## Tabelle 2*

## Wirkung der Peptide auf die Aktivierung des Faktor X

| Inhibitor | Hemmkonstante |
|---|---|
| Asp-Gly-Asp-Gln-Cys | 105 µM |
| Lys-Gln-Tyr-Val-Asp-Gly-Asp-Gln-Cys | 108 µM |
| Lys-Gln-Tyr-Val-Asp-Gly-Asp-Gln-Cys-Glu-Ser-Asn-NH$_2$ | 100 µM |
| Lys-Gln-Tyr-Val-Asp-Gly-Asp-Gln-NH$_2$ | 115 µM |
| Asp-Gly-Tyr-Arg-Leu-Ala-Glu-Asp-Gln-Lys-Ser-Cys-NH$_2$ | >2 mM |
| Lys-Asn-Gly-Arg-Cys-Lys-Gln-Phe-Cys-Lys-Arg-Asp-Thr-Asp-Asn-Lys-NH$_2$ | >2 mM |

* Inhibierung der Faktor X Aktivierung über Einzellschichten von Endothelzellen durch die Peptide wurde wie im Text beschrieben durchgeführt. Die Hemmkonstante ist als Mittelwert der experimentell bestimmten Werte angegeben. Der Standardfehler des Mittelwertes betrug weniger als 20%.

Die Daten in Tabelle 2 zeigen, dass alle Peptide die Aktivierung des Faktor X inhibieren, ausser das Dodecapeptid, das den Aminosäureresten 91-106 des Faktors IX vom Rind entspricht, und das Hexadecapeptid. Keines dieser Peptide hatte bis zu einer Konzentration von 2 mM eine Wirkung auf die Faktor Xa Bildung. Das Decapeptid wirkte auch als ein Inhibitor der Faktor X Aktivierung (Daten nicht gezeigt). Die Aehnlichkeit der Werte der Inhibitorkonstanten für die Faktor IX-Bindung (Tabelle 1) mit denen der kinetischen Studien (Tabelle 2) unterstützt die Hypothese, dass Inhibierung der Bindung von Faktor IX/IXa an Endothelzellen die Wirkungsweise der Peptide darstellt. Uebereinstimmend mit dieser Annahme ist die fehlende Inhibierung der Faktor X-Aktivierung, durch das modifizierte Pentapeptid Glu-Gly-Glu-Gln-Cys (Daten nicht gezeigt).

In vivo Studie: Auf der Grundlage der Ergebnisse obiger in vitro-Studien, die zeigen, dass einige der "EGF-Domänen"-Peptide die Wechselwirkung von Faktor IX/IXa mit Endothelzellen blockieren können, wurden in vivo Studien mit diesen Peptiden durchgeführt. Als in vivo Modell wurde das Wesslersche Venostase-Thrombose-Modell [Gitel et al. Proc. Natl. Acad. Sci. USA 74, 3028-3032 (1977)] gewählt, wobei als Prokoagulationsmittel der Faktor IXa (600 ng) verwendete wurde. Das Verfahren wurde in mit Natriumpentabarbital narkotisierten Kaninchen durch Injektion von Rinderfaktor IXa alleine oder in Gegenwart von 10 mg des Pentapeptids Asp-Gly-Asp-Gln-Cys durchgeführt. Ein Segment der exponierten contralateralen Jugularisvene, wurde nach 10 Sekunden unterbunden. Nach 16 Minuten wurde das isolierte Gefäss in 5% (Gewicht/Volumen) Natriumcitrat gegeben und geöffnet.

Die Thromben wurden nach einer Skala von 0-4, wie bei Gitel et al. (supra), eingeteilt. Tiere, die mit Faktor IXa alleine injiziert worden waren, zeigten Thromben des Grades 3, wobei die mit Faktor IXa und Peptid behandelten Tiere nur Thromben des Grades 1 zeigten. In einem Experiment, in dem 8 mg Pentapeptid ohne Faktor IXa in Kaninchen injiziert worden war, waren die Prothrombinzeit und die aktivierte partielle Thromboplastinzeit von citratgepufferten Blutproben (1, 3, 5 und 10 Minuten nach Infusion des Peptids gesammelt) unverändert.

In ähnlichen Studien, in denen das aktive Dodecapeptid verwendet wurde, lieferten Peptidgaben von 100, 70 und 50 mg Thromben des Grades 0, 1 und 1. Die Prothrombinzeit und die aktivierte partielle Thromboplastinzeit citratgepufferter Blutproben von Tieren, die eine Dosis von 70 mg des Dodecapeptides allein erhalten hatten, waren dann immer noch unverändert.

Die Ergebnisse legen den Schluss nahe, dass die erfindungsgemässen Peptide der EGF-Domäne einen neuen Typ von Therapeutika mit antithrombotischer Wirkung darstellen; sie können intravaskuläre Thrombosebildung blockieren ohne die Blutgerinnung in vitro zu verändern.

## Ansprüche

1. Ein zur selektiven Bindung an die zellulären Rezeptoren des Faktors IX/IXa befähigtes Peptid, dadurch gekennzeichnet, dass es die EGF-Domäne des Faktors IX oder eine Untersequenz davon enthält.

2. Ein Peptid gemäss Anspruch 1 mit der Aminosäuresequenz Asp-Gly-Asp-Gln-Cys.

3. Ein Peptid gemäss Anspruch 1 mit der Aminosäuresequenz Lys-Gln-Tyr-Val-Asp-Gly-Asp-Gln-Cys.

4. Ein Peptid gemäss Anspruch 1 mit der Aminosäuresequenz Lys-Gln-Tyr-Val-Asp-Gly-Asp-Gln-Cys-Glu-Ser-Asn-NH2.

5. Ein Peptid gemäss Anspruch 1 mit der Aminosäuresequenz Lys-Gln-Try-Val-Asp-Gly-Asp-Gln-NH2.

6. Ein Peptid gemäss Anspruch 1 mit der Aminosäuresequenz Phe-Trp-Lys-Gln-Tyr-Val-Asp-Gly-Asp-Gln-NH2.

7. Ein Peptid gemäss einem der Ansprüche 1-6 zur Behandlung thrombotischer Erkrankungen.

8. Ein Verfahren zur Herstellung eines Peptids gemäss einem der Ansprüche 1-6, dadurch gekennzeichnet, dass konventionelle Methoden der Peptidsynethese verwendet werden.

9. Ein Verfahren gemäss Anspruch 8, dadurch gekennzeichnet, dass eine Festphasensynthesemethode verwendet wird.

10. Eine pharmazeutische Zusammensetzung, die ein Peptid gemäss einem der Ansprüche 1-7 und ein pharmazeutisch sinnvolles Trägermaterial enthält.

11. Verwendung eines Peptides gemäss einem der Ansprüche 1-6 zur Behandlung thrombotischer Erkrankungen.

12. Verwendung eines Peptides gemäss einem der Ansprüche 1-7 zur Herstellung einer pharmazeutischen Zusammensetzung gemäss Anspruch 10.

## EUROPÄISCHER TEILRECHERCHENBERICHT,

**Europäisches Patentamt**

der nach Regel 45 des Europäischen Patentübereinkommens für das weitere Verfahren als
europäischer Recherchenbericht gilt

Nummer der Anmeldung

EP 87 11 4793

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | NATURE, Band 307, 9. Februar 1984, Seiten 558-60 Macmillan Journals Ltd, GB; R.F. DOOLITTLE et al.: "Computer-based characterization of epidermal growth factor precursor" <br><br> * Seite 558, linke Spalte, rechte Spalte, Zeilen 1-3; Seite 559; Figur 2; Seite 560, Zeilen 37-44 * | 1,12 | C 07 K 7/06 <br> C 07 K 7/08 |
| | ------------------------- | | |

### RECHERCHIERTE SACHGEBIETE (Int. Cl.4)

C 07 K 7/00
A 61 K 35/00
A 61 K 37/00

### UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-10,12

Unvollständig recherchierte Patentansprüche:

Nicht recherchierte Patentansprüche: 11

Grund für die Beschränkung der Recherche:

Verfahren zur chirurgischen oder therapeutischen Behandlung des menschlichen oder tierischen Körpers (siehe Art. 52(4) des Europäischen Patentübereinkommens).

| Recherchenort | Abschlußdatum der Recherche | Prüfer |
|---|---|---|
| Den Haag | 26-01-1988 | MASTURZO |